# FASCICULE DE BREVET EUROPEEN

(11) **EP 1 486 199 B1**
(45) Date de publication et mention de la délivrance du brevet: **17.10.2007**
(21) Numéro de dépôt: 04290958.0
(22) Date de dépôt: 09.04.2004
(51) Int. Cl.: A61Q 15/00, A61K 8/27, A61K 8/34, A61K 8/368, A61K 8/31

(54) **Composition déodorante aérosol alcoolique contenant le salicylate de zinc**
Desodorierende alkoholische Aerosolzusammensetzung enthaltend Zinksalicylat
Alcoholic aerosol deodorant composition containing zinc salicylate

(30) Priorité: 02.06.2003 FR 0306622
(43) Date de publication de la demande: 15.12.2004
(73) Titulaire: L'ORÉAL, 75008 Paris (FR)
(72) Inventeur: Lemoine, Cyril, 95380 Puiseux-en-France (FR)
(74) Mandataire: Miszputen, Laurent

(56) Documents cités:
- EP-A- 0 318 206
- EP-A- 0 468 564
- US-A- 5 302 381
- US-B1- 6 344 218

## Description

La présente invention a trait à une composition déodorante caractérisée par le fait qu'elle contient dans un véhicule cosmétiquement acceptable :
(a) au moins le salicylate de zinc, sous forme libre ou hydratée;
(b) au moins un mono-alcool en C₁-C₄;
(c) au moins un agent propulseur.

L'invention concerne également un dispositif aérosol constitué par un récipient comprenant une composition aérosol constituée, d'une part par une phase liquide (ou jus) comprenant au moins un mono-alcool en C₁-C₄ et au moins le salicylate de zinc, sous forme libre ou hydratée et au moins un agent propulseur et par un moyen de distribution de la dite composition aérosol.

L'invention concerne également un procédé de traitement des odeurs corporelles humaines et notamment axillaires à l'aide de ce dispositif.

Dans le domaine cosmétique, il est bien connu d'utiliser, en application topique, des produits déodorants contenant des substances actives, de type antitranspirant ou de type bactéricide, pour diminuer, voire supprimer, les odeurs axillaires généralement désagréables.

Les substances antitranspirantes ont pour effet de limiter le flux sudoral. Elles sont généralement constituées de sels d'aluminium qui, d'une part, sont irritants pour la peau et qui, d'autre part, diminuent le flux sudoral en modifiant la physiologie cutanée, ce qui n'est pas satisfaisant.

Les substances bactéricides inhibent le développement de la flore cutanée responsable des odeurs axillaires.

Parmi les produits bactéricides, le plus couramment employé est le Triclosan (2,4,4'-trichloro-2'-hydroxydlphényléther), qui présente inconvénient de modifier de façon importante l'écologie de la flore cutanée. On connaît également le 3,7,11-triméthyldodéca-2,5,10-triénol (Farnesol) qui présente l'inconvénient d'être allergène.

Dans le but d'obtenir une efficacité à long terme, on recherche de nouveaux produits exerçant une action déodorante, c'est-à-dire des produits qui soient capables de modifier, de réduire et/ou d'ôter ou de prévenir le développement des odeurs corporelles (cette définition est donnée dans l'ouvrage « Cosmetic Science and Technology Series » - 1988 / Volume 7 chap.10 - IIIc). En outre, on recherche des produits qui ne présentent pas les inconvénients des substances actives utilisées dans l'art antérieur.

Dans cette optique, à titre d'actif déodorant, certains sels hydrosolubles de zinc ont déjà été proposés dans des produits déodorants comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc. De telles formulations déodorantes ont été notamment décrites dans les documents WO93/01793, EP468564, EP024176 et EP768080. Cependant, de tels absorbeurs d'odeurs à base de zinc sont peu satisfaisants du fait qu'ils sont pour la plupart difficilement formulables dans der aérosols alcooliques qui représentent aujourd'hui la majorité des produits déodorants sur le marché.

Dans les années 1930 comme le décrit en particulier l'ouvrage Drugs & Cosmetic Industry-Modern Cosmetics ; Chapître Déodorants pages 168-177 (1934), on savait que le salicylate de zinc pouvait être formulé dans des formulations déodorantes du type pâte ou sous forme de stick en présence nécessaire d'autres actifs pour une action déodorante suffisante. Il a été proposé plus récemment comme actif déodorant possible dans différents supports : selon le brevet EP318206 dans des gels sticks à base de benzylidene sorbitol, d'un émollient du type di-isopropyladlpate, d'un ou plusieurs solvants polaires et d'un agent de couplage ; selon les brevets US 5,254,332 et US 5,302,381 dans des sticks contenant un huile siliconée volatile, une cire de bas point de fusion, un émollient Insoluble dans l'eau liquide et en présence d'un actif déodorant du type bicarbonate de sodium ; selon la demande WO02/13776 dans des gels aqueux à base de savons d'acides gras.

Après de nombreuses recherches menées sur la question, la Demanderesse a maintenant découvert, de façon inattendue et surprenante, que d'une part le salicylate de zinc présentait un spectre d'activité bactéricide sensiblement moins large que celui du Triclosan et respectait donc d'avantage la flore cutanée et que, d'autre part, on pouvait le formuler dans un aérosol alcoolique classique et obtenir une formulation stable au stockage et en fin qu'il avait une bonne efficacité déodorante.

Cette découverte est à la base de la présente invention.

Le salicylate de zinc selon l'invention répond à la structure suivante : 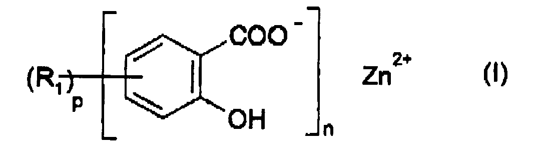 dans laquelle n = 2, p vaut 0.

On choisira plus préférentiellement le salicylate de zinc sous forme hydratée comme le salicylate de zinc trihydraté tel que le produit commercial vendu sous le "Zinc Salicylate Trihydrate" par la société Bernardy Chimie.

Dans les compositions de l'invention, la concentration en salicylate de zinc varie de préférence de 0,001 à 20%, plus préférentiellement de 0,01 à 15% et encore plus préférentiellement de 0,05 à 5% en poids par rapport au poids total de la composition.

Le ou les mono-alcools en C₁-C₄ présents dans les compositions de l'invention peuvent être choisis parmi le méthanol, l'éthanol, l'isopropanol ou leurs mélanges. On choisira plus particulièrement l'éthanol. Ils sont en général présents en général à des concentrations allant de 15 à 80 % en poids, plus préférentiellement de 30 à 70 % en poids et encore plus préférentiellement 40 à 70 % en poids par rapport au poids total de la composition.

L'agent propulseur peut être choisi parmi le diméthyléther ; les hydrocarbures volatils tels que le n-butane, le propane, l'isopropane, le n-butane, l'isobutane, le pentane et l'isopentane et leurs mélanges ; les hydrocarbures volatils fluorés tels que le 1,1-difluoroéthane et le 1,1,1-trifluoro-2-fluoro éthane. On peut également utiliser en tant qu'agent propulseur le gaz carbonique, le protoxyde d'azote, l'azote ou l'air comprimé.

La composition contenant le salicylate de zinc et le ou les agents propulseurs peuvent se trouver dans le même compartiment ou dans des compartiments différents dans le récipient aérosol.

Selon l'invention, la concentration en agent propulseur varie généralement de 5 à 90% en poids et plus préférentiellement de 15 à 70% en poids par rapport au poids total de la composition pressurisée.

En plus du salicylate de zinc, la composition selon l'invention peut également contenir d'autres actifs déodorants ou des actifs antl-transplrants additionnels.

Parmi les actifs déodorants additionnels, on peut citer par exemple : le pyrrolidone carboxylate de zinc (plus communément appelé pidolate de zinc), le sulfate de zinc, le chlorure de zinc, le lactate de zinc, le gluconate de zinc et le phénolsulfonate de zinc, le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Trielocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

Les actifs préférés sont 2,4,4'-trichloro-2'-hydroxydiphényléther (Triclosan) ; les sels de polyhexaméthylène biguanide (connu sous le nom de sels de polyaminopropyl biguanide) - par exemple le produit Cosmocil CQTM (Zeneca) ; le 3,7,11-triméthyldodéca-2,5,10-triénol (Farnesol).

Parmi les actifs anti-transpirants additionnels, on peut citer par exemple le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate, les complexes de métaux (comme l'aluminium ou le zirconium) avec un acide aminé et tels que décrits dans le brevet US-3792068. De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/CI allant d'environ 0,73 à 1,93.

L'amino-acide préféré pour préparer de tels complexes ZAG est la glycine. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Ces actifs déodorants ou anti-transpirants additionnels peuvent être présents dans la composition selon l'invention à raison d'environ 0,001 à 20% en poids par rapport à la composition totale, et de préférence à raison d'environ 0,1 à 10% en poids.

Selon une forme particulière de l'invention, la composition déodorante aérosol alcoolique ne contiendra pas d'actif déodorant ou anti-transpirant additionnel tel que ceux cités précédemment.

Le véhicule cosmétiquement acceptable qui est essentiellement alcoolique peut contenir de l'eau et/ou un ou plusieurs solvants organiques additionnels comme le propylène glycol, le dipropylène glycol ou leurs éthers.

La composition cosmétique selon l'invention peut également être formulée en émulsion eau-dans-huile, huile-dans-eau, ou en émulsion triple eau-dans-huile-dans-eau, (de telles émulsions sont connues et décrites par exemple par C. FOX dans « Cosmetics and Toiletries » - November 1986 - Vol 101 - pages 101-112).

La composition cosmétique selon l'invention peut comprendre en outre des adjuvants cosmétiques choisis parmi les corps gras, les émollients, les adoucissants, les antioxydants, les opacifiants, les stabilisants, les agents anti-mousse, les agents hydratants, les vitamines, les parfums, les conservateurs, les tensioactifs, les charges, les séquestrants, les polymères, les agents alcalinisants
ou acidifiants, les parfums, les colorants, les pigments, les agents épaississants, ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les tensio-actifs sont choisis de préférence parmi les tensio-actifs anioniques, amphotères ou non-ioniques.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en C₁₂-C₁₅ (Finsolv TN de FINETEX), l'alcool myristique polyoxypropyléné à 3 moles d'oxyde de propylène (WITCONOL APM de WITCO), les triglycérides d'acides gras en C6-C18 tels que les triglycérides d'acide caprylique/caprique.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin (octanoate de stéaryle), les huiles de silicone et les isoparaffines.

Les cires sont choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

La composition cosmétique peut comprendre des émollients, qui contribuent à une sensation douce, sèche, non-collante à l'application de la composition sur la peau. Ces émollients peuvent être choisis parmi des produits du type silicone volatile, des silicones non-volatiles et d'autres émollients non-volatils.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4 ou D5.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 200 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les émollients non-volatils utilisables dans la présente invention, on peut citer par exemple : les dérivés hydrocarbonés, les huiles minérales, les alcools gras, les esters d'alcools en C₃-C₁₈ avec des acides en C₃-C₁₈, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polalkylène glycol.

Les quantités de ces différents constituants pouvant être présents dans la composition cosmétique selon l'invention sont celles classiquement utilisées pour les formes de présentation considérées.

La composition cosmétique selon l'invention peut ainsi se présenter sous forme de lotion, de crème ou de gel fluide distribués en spray aérosol, et contenir à cet égard les ingrédients généralement utilisés dans ce type de produits et bien connus de l'homme de l'art.

Le moyen de distribution, qui forme une partie du dispositif aérosol, est généralement constitué par une valve de distribution commandée par une tête de distribution, elle même comprenant une buse par laquelle la composition aérosol est vaporisée.

Le récipient contenant la composition pressurisée peut être opaque ou transparent.

Il peut être en verre, en matériau polymérique ou en métal, recouvert éventuellement d'une couche de vernis protecteur.

Des exemples concrets, mais nullement limitatif, illustrant l'invention vont maintenant être donné.

On réalise le dispositif aérosol suivant conforme à l'invention en introduisant le jus suivant et les propulseurs dans un récipient aérosol munis des moyens de distribution classiques

| **Exemple 1** | **quantités** |
|---|---|
| Salicylate de zinc trihydraté | 0.99 g |
| Isobutane | 55,0 g |
| Alcool éthylique 96 °dénaturé | 44,1 g |

La formule aérosol alcoolique obtenue est transparente et stable après 2 mois de stockage à 45°C.

### Test d'efficacité

Des recueils de sueur axillaire sont effectués en sauna sur 6 volontaires, les échantillons de sueur individuelle, conservés dans la glace pendant quelques heures, sont alors pratiquement inodores. Il sont ensuite mélangés et répartis en aliquots de 1 ml. Les actifs sont rajoutés à ces aliquots, puis mis à incuber à l'étuve 37°C. Après 18 et 24 heures d'incubation, un jury d'experts évalue l'intensité de l'odeur comparativement à un échantillon témoin : 1 ml de sueur incubée sans ajout d'actif.

Les résultats sont exprimés en % de variation du désagrément de l'odeur comparativement à cet échantillon de sueur témoin (moyenne des % de variation à T18h et T24h).

| | (A) | (B) | (C) |
|---|---|---|---|
| Quantité testée (mg MA/ml de sueur) | 1 mg | 5mg | 0.8mg |
| % variation du désagrément d'odeur | - 45 % | - 48% | 55 % |

Les actifs (A) et (B) correspondent au salicylate de zinc trihydraté de Bernardy Chimie et l'actif (C) au Triclosan.

### MISE EN EVIDENCE DE L'ACTIVITE BACTERICIDE D'UNE COMPOSITION VIS-A-VIS DES MICRO-ORGANISMES IMPLIQUES DANS LES ODEURS AXILLAIRES

Le test décrit ici permet la détermination quantitative de l'activité bactéricide d'une composition sur des germes en conditions optimales de croissance à savoir des germes du type Corynebacterium xerosis (Collection de l'Institut Pasteur n° 5216) cultivés sur gélose Tryptocaséine soja en pente.

La veille du test, dans un pilulier, on dépose 32 g de bouillon Tryptocaséine soja et l'on met à incuber à 35°C. Le jour du test, on ajoute 4 g de la composition à tester et on homogénéise au Vortex.

Un témoin de croissance sans produit est préparé dans les mêmes conditions afin de vérifier que les germes sont dans des conditions de croissance favorables pendant toute la durée du test.

Pour la préparation de l'inocumum, cinq jours avant le début du test, on pratique un repiquage des deux souches bactériennes sur milieu approprié. On incube 5 jours à 35°C. Le jour du test, on lave la pente avec environ 9 ml de diluant: La suspension obtenue titre à 108 germes/ml (on effectue un dénombrement). On introduit 4 ml d'inoculum dans le pilulier, ce qui correspond à un taux de 107 bactéries par gramme de préparation. On place le pilulier dans un incubateur-agitateur (35°C - 200 RPM).

Après chaque temps de contact (2, 4, 6 et 24 heures), on homogénéise le contenu du pilulier au Vortex. On effectue des dilutions décimales. On dépose en surface de boîtes de Pétri gélosée (milieu Eugon LT 100). On incube les boîtes de Pétri 6 à 7 jours à l'étuve à 35°C.

On procède au comptage des colonies sur les boîtes contenant plus de 20 et moins de 200 colonies.

En ce qui concerne l'évaluation en tant qu'actif, les résultats sont exprimés, pour chaque micro-organisme testé, en nombre de Log de réduction au bout de 24 heures par rapport au placebo :

| | |
|---|---|
| résultats identiques : | nulle, |
| 1 Log de réduction : | faible, |
| 2 Log de réduction : | moyenne, |
| 3 Log de réduction : | bonne, |
| ≥ 4 Log de réduction : | excellente. |

On a réalisé les trois formulations suivantes :

| **Ingrédients** | **Formule 2 (invention)** | **Formule 3 (invention)** | **Formule 4 (art antérieur)** |
|---|---|---|---|
| Triclosan | | | 0,1g |
| Salicylate de zinc | 0.1 g | 1 g | |
| Polyéthyleneglycol à 8 OE | | | 6g |
| Copolymère réticulé acide acrylique/acrylate d'alkyle en C₁₀-C₃₀ | | | 0.9g |
| Hydroxyde de sodium pur | | | qs (pH=7) |
| Eau désionisée microbiologiquement propre | 99.9 g | 99 g | 93.0 g |

On mesure l'activité bactéricide de chacune des formulations 2, 3 et 4 vis à vis de la souche Corynebacterium xerosis et on l'a compare avec une formule sans actif.

Les résultats obtenus sont résumés dans le tableau suivant :

| **Compositions** | **Efficacité à 24 heures par rapport au gel sans actif** |
|---|---|
| Souches | Corynebacterium xerosis |
| 2 (invention) | Faible |
| 3 (invention) | Faible |
| 4 (art antérieur) | Excellent |

Le salicylate de zinc a une activité antibactérienne faible sur les souches Corynebacterium Xerosis. Il a donc un spectre d'activité bactéricide sensiblement moins large que celui du Triclosan et respecte d'avantage la flore cutanée

## Revendications

1. Composition déodorante **caractérisée par le fait qu'**elle contient dans un véhicule cosmétiquement acceptable:
(a) au moins le salicylate de zinc, sous forme libre ou hydratée;
(b) au moins un mono-alcool en C₁-C₄;
(c) au moins un agent propulseur.

2. Composition selon la revendication 1, où le salicylate de zinc est le salicylate de zinc trihydraté.

3. Composition selon l'une quelconque des revendications 1 ou 2, **caractérisée par le fait que** la concentration en salicylate de zinc varie de 0.001 à 20%, de préférence de 0,01 à 15% et encore préférentiellement de 0,05 à 5% en poids par rapport au poids total de la composition.

4. Composition selon l'une quelconque des revendications 1 à 3, **caractérisée par le fait que** le ou les mono-alcools en C₁-C₄ sont choisis parmi le méthanol, l'éthanol, l'isopropanol ou leurs mélanges.

5. Composition selon l'une quelconque des revendications 1 à 4, **caractérisée par le fait que** le mono-alcool en C₁-C₄ est l'éthanol.

6. Composition selon l'une quelconque des revendications 1 à 5 **caractérisée par le fait que** le ou les mono-alcools en C₁-C₄ sont présents à des concentrations allant de 15 à 80 % en poids, plus préférentiellement de 30 à 70 % en poids et encore plus préférentiellement de 40 à 70 % en poids par rapport au poids total de la composition.

7. Composition selon l'une quelconque des revendications 1 à 6, **caractérisée par le fait que** l'agent propulseur est choisi parmi le diméthyléther, les hydrocarbures volatils, les hydrocarbures volatils fluorés et leurs mélanges.

8. Composition selon la revendications 7, où le ou les hydrocarbures volatils sont choisis parmi le propane, l'isopropane, le n-butane, l'isobutane, le pentane, l'isopentane et leurs mélanges.

9. Composition selon la revendication 7, où le ou les hydrocarbures volatils fluorés sont choisis parmi le 1,1-difluoroéthane, le 1,1,1-trifluoro-2-fluoro éthane et leurs mélanges.

10. Composition selon l'une quelconque des revendications 1 à 6, **caractérisée par le fait que** l'agent propulseur est choisi parmi le gaz carbonique, le protoxyde d'azote, l'azote ou l'air comprimé.

11. Composition selon l'une quelconque des revendications 1 à 10, **caractérisée par le fait que** la concentration en agent propulseur varie de 5 à 90% en poids et plus préférentiellement de 15 à 70% en poids par rapport au poids total de la composition pressurisée.

12. Composition selon l'une quelconque des revendications 1 à 11, **caractérisée par le fait qu'**elle contient en plus un ou plusieurs actifs déodorants et/ou actifs anti-transpirants additionnels.

13. Composition selon la revendication 12, où les actifs déodorants additionnels sont choisis parmi le pyrrolidone carboxylate de zinc ; le sulfate de zinc ; le chlorure de zinc ; le lactate de zinc ; le gluconate de zinc ; le phénolsulfonate de zinc ; le 2,4,4'-trichloro-2'-hydroxydiphényléther (Triclosan) ; le 2,4-dichloro-2'-hydroxydiphényléther ; le 3',4',5'-trichiorosallcylanilide ; la 1-(3',4'-dichlorophenyl)-3-(4'-chlorophenyl)urée (Triclocarban); le 3,7,11-triméthyldodéca-2,5,10-triénol (Farnesol); les sels de cetyltrimethylammonium; les sels de cétylpyridinium ; la chlorhexidine et ses sels; le monocaprate de diglycérol; le monolaurate de diglycérol, le monolaurate de glycérol ; les sels de polyhexaméthylène biguanide.

14. Composition selon la revendication 12, où les actifs ant-transpirants sont choisis parmi le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate, les complexes de métaux avec un acide aminé.

15. Composition selon la revendication 14, **caractérisé par le fait que** l'acide aminé utilisé avec les complexes de métaux est la glycine.

16. Composition selon l'une quelconque des revendications 12 à 15, **caractérisée par** le fait les actifs déodorants et/ou antitranspirants additionnels sont présents à raison d'environ 0,001 à 20% en poids par rapport à la composition totale, et de préférence à raison d'environ 0,1 à 10% en poids.

17. Composition selon l'une quelconque des revendications 1 à 11, **caractérisée par le fait qu'**elle ne contient pas d'actif déodorant ou anti-transpirant additionnel.

18. Composition selon l'une quelconque des revendications 1 à 17, **caractérisée par le fait que** le véhicule cosmétiquement acceptable contient en plus de l'eau et/ou un ou plusieurs solvants organiques additionnels.

19. Composition selon l'une quelconque des revendications 1 à 18, **caractérisée par le fait qu'**elle est formulée en émulsion eau-dans-huile, huile-dans-eau, ou en émulsion triple eau-dans-huile-dans-eau.

20. Composition selon l'une quelconque des revendications 1 à 19, **caractérisée par le fait qu'**elle comprend en outre un ou plusieurs adjuvants cosmétiques choisis parmi les corps gras, les émollients, les adoucissants, les antloxydants, les opacifiants, les stabilisants, les agents anti-mousse, les agents hydratants, les vitamines, les parfums, les conservateurs, les tensioactifs, les charges, les séquestrants, les polymères, les agents alcalinisants ou acidifiants, les parfums, les colorants, les pigments, les agents épaississants, ou tout autre ingrédient habituellement utilisé en cosmétique pour ce type d'application.

21. Composition selon l'une quelconque des revendications 1 à 20, **caractérisée par le fait qu'**elle se présente sous forme de lotion, de crème ou de gel fluide.

22. Dispositif aérosol constitué par un récipient comprenant une composition aérosol constituée, d'une part par une phase liquide (ou jus) comprenant au moins un mono-alcool en C₁-C₄ et le salicylate de zinc, sous forme libre ou hydratée et au moins un agent propulseur tels que définis dans l'une quelconque des revendications précédente et par un moyen de distribution de la dite composition aérosol.

23. Dispositif selon la revendication 22, **caractérisé par le fait que** le salicylate de zinc le ou les mono-alcools et le ou les agents propulseurs peuvent se trouver dans le même compartiment ou dans des compartiments différents dans le récipient aérosol.

24. Procédé de traitement des odeurs corporelles humaines et notamment axillaires par utilisation du dispositif décrit selon l'une quelconque des revendications 22 ou 23.

## Claims

1. Deodorant composition, **characterized in that** it contains, in a cosmetically acceptable vehicle:
(a) at least zinc salicylate, in free or hydrated form;
(b) at least one C₁-C₄ monoalcohol;
(c) at least one propellant.

2. Composition according to Claim 1, in which the zinc salicylate is zinc salicylate trihydrate.

3. Composition according to either of Claims 1 and 2, **characterized in that** the zinc salicylate concentration ranges from 0.001% to 20%, preferably from 0.01% to 15% and more preferably from 0.05% to 5% by weight relative to the total weight of the composition.

4. Composition according to any one of Claims 1 to 3, **characterized in that** the C₁-C₄ monoalcohol(s) is (are) chosen from methanol, ethanol and isopropanol, or mixtures thereof.

5. Composition according to any one of Claims 1 to 4, **characterized in that** the C₁-C₄ monoalcohol is ethanol.

6. Composition according to any one of Claims 1 to 5, **characterized in that** the C₁-C₄ monoalcohol(s) is (are) present at concentrations ranging from 15% to 80% by weight, more preferably from 30% to 70% by weight and even more preferably from 40% to 70% by weight relative to the total weight of the composition.

7. Composition according to any one of Claims 1 to 6, **characterized in that** the propellant is chosen from dimethyl ether, volatile hydrocarbons and volatile fluorohydrocarbons, and mixtures thereof.

8. composition according to Claim 7, in which the volatile hydrocarbons are chosen from propane, isopropane, n-butane, isobutane, pentane and isopentane, and mixtures thereof.

9. Composition according to Claim 7, in which the volatile fluorohydrocarbon(s) is (are) chosen from 1,1-difluoroethane and 1,1,1-trifluoro-2-fluoroethane, and mixtures thereof.

10. Composition according to any one of Claims 1 to 6, **characterized in that** the propellant is chosen from carbon dioxide, nitrous oxide, nitrogen and compressed air.

11. Composition according to any one of Claims 1 to 10, **characterized in that** the propellant concentration ranges from 5% to 90% by weight and more preferably from 15% to 70% by weight relative to the total, weight of the pressurized composition.

12. Composition according to any one of Claims 1 to 11, **characterized in that** it also contains one or more additional deodorant active agents and/or antiperspirant active agents.

13. Composition according to Claim 12, in which the additional deodorant active agents are chosen from zinc pyrrolidonecarboxylate; zinc sulphate; zinc chloride; zinc lactate zinc gluconate; zinc phenolsulphonate; 2,4,4'-trichloro-2'-hydroxydiphenyl ether Triclosan); 2,4-dichloro-2'-hydroxydiphenyl ether; 3',4',5'-trichlorosalicylanilide; 1-(3' 4'-dichlorophenyl)-3-(4'-chlorophenyl)urea (Triclocarban) ; 3,7,11-trimethyldodeca-2,5,10-trienol (Farnesol); cetyltrimethylammonium salts; cetylpyridinium salts; chlorhexidine and salts thereof; diglyceryl monocaprate; diglyceryl monolaurate; glyceryl monalaurate; polyhexamethylene biguanide salts.

14. Composition according to Claim 12, in which the antiperspirant active agents are chosen from aluminium chlorohydrate aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG aluminium sulphate, aluminium zirconium octachlorohydrate, aluminium zirconium pentachlorohydrate, aluminium zirconium tetrachlorohydrate, aluminium zirconium trichlorohydrate, complexes of metals with an amino acid.

15. Composition according to Claim 14, **characterized in that** the amino acid used with the metal complexes is glycine.

16. Composition according to any one of Claims 12 to 15, **characterized in that** the additional deodorant and/or antiperspirant active agents are present in a proportion of about from 0.001% to 20% by weight and preferably in a proportion of about from 0.1% to 10% by weight relative to the total composition.

17. Composition according to any one of Claims 1 to 11, **characterized in that** it contains no additional deodorant or antiperspirant active agent.

18. Composition according to any one of Claims 1 to 17, **characterized in that** the cosmetically acceptable vehicle also contains water and/or one or more additional organic solvents.

19. Composition according to any one of Claims 1 to 18, **characterized in that** it is formulated as a water-in-oil or oil-in-water emulsion, or as a water-in-oil-in-water triple emulsion.

20. Composition according to any one of Claims 1 to 19, **characterized in that** it also comprises one or more cosmetic adjuvants chosen from fatty substances, emollients, softeners, antioxiciants, opacifiers, stabilizers, antifoams, moisturizers, vitamins, fragrances, preserving agents, surfactants, fillers, sequestering agents, polymers, acidifying or basifying agents, fragrances, dyes, pigments and thickeners, or any other ingredient usually used in cosmetics for this type of application.

21. Composition according to any one of claims 1 to 20, **characterized in that** it is in the form of a lotion, a cream or a fluid gel.

22. Aerosol device consisting of a container comprising an aerosol composition constituted tirstly of a liquid phase (or fluid) comprising at least one C₁-C₄ monoalcohol and zinc salicylate, in free or hydrated form, and at least one propellant as defined in any one of the preceding claims, and of a means for distributing the said aerosol composition.

23. Device according to Claim 22, **characterized in that** the zinc salicylate, the monoalcohol(s) and the propellant(s) may be in the same compartment or in different compartments in the aerosol container.

24. Process for treating human body odour, and especially underarm odour, by using the device described according to either of Claims 22 and 23.

## Patentansprüche

1. Desodorierende Zusammensetzung, **dadurch gekennzeichnet, dass** sie in einem kosmetisch akzeptablen Träger enthält:
(a) zumindest Zinksalicylat in freier Form oder in hydratisierter Form;
(b) mindestens einen C₁₋₄-Monoalkohol;
(c) mindestens ein Treibmittel.

2. Zusammensetzung nach Anspruch 1, wobei das Zinksalicylat das Zinksalicylat-Trihydrat ist.

3. Zusammensetzung nach einem der Ansprüche 1 oder 2, **dadurch gekennzeichnet, dass** die Konzentration des Zinksalicylats im Bereich von 0,001 bis 20 Gew.-%, vorzugsweise 0,01 bis 15 Gew.-% und noch bevorzugter 0,05 bis 5 Gew.-%, bezogen auf das Gesamtgewicht der Zusammensetzung, liegt.

4. Zusammensetzung nach einem der Ansprüche 1 bis 3, **dadurch gekennzeichnet, dass** der oder die C₁₋₄-Monoalkohole unter Methanol, Ethanol, Isopropanol oder deren Gemischen ausgewählt sind.

5. Zusammensetzung nach einem der Ansprüche 1 bis 4, **dadurch gekennzeichnet, dass** es sich bei dem C₁₋₄-Monoalkohol um Ethanol handelt.

6. Zusammensetzung nach einem der Ansprüche 1 bis 5, **dadurch gekennzeichnet, dass** der oder die C₁₋₄-Monoalkohole in Konzentrationen von 15 bis 80 Gew.-%, noch bevorzugter 30 bis 70 Gew.-% und besonders bevorzugt 40 bis 70 Gew.-%, bezogen auf das Gesamtgewicht der Zusammensetzung, enthalten sind.

7. Zusammensetzung nach einem der Ansprüche 1 bis 6, **dadurch gekennzeichnet, dass** das Treibmittel unter Dimethylether, flüchtigen Kohlenwasserstoffen, flüchtigen fluorierten Kohlenwasserstoffen und deren Gemischen ausgewählt ist.

8. Zusammensetzung nach Anspruch 7, **dadurch gekennzeichnet, dass** der oder die flüchtigen Kohlenwasserstoffe unter Propan, Isopropan, n-Butan, Isobutan, Pentan, Isopentan und deren Gemischen ausgewählt sind.

9. Zusammensetzung nach Anspruch 7, wobei der oder die flüchtigen fluorierten Kohlenwasserstoffe unter 1,1,-Difluorethan, 1,1,1-Trifluor-2-fluorethan und deren Gemischen ausgewählt sind.

10. Zusammensetzung nach einem der Ansprüche 1 bis 6, **dadurch gekennzeichnet, dass** das Treibmittel unter Kohlendioxid, Distickstoffoxid, Stickstoff oder Druckluft ausgewählt ist.

11. Zusammensetzung nach einem der Ansprüche 1 bis 10, **dadurch gekennzeichnet, dass** die Konzentration des Treibmittels im Bereich von 5 bis 90 Gew.-% und bevorzugt 15 bis 70 Gew.-%, bezogen auf das Gesamtgewicht der unter Druck stehenden Zusammensetzung liegt.

12. Zusammensetzung nach einem der Ansprüche 1 bis 11, **dadurch gekennzeichnet, dass** sie ferner einen oder mehrere desodorierende Wirkstoffe und/oder schweißverhütende Wirkstoffe enthält.

13. Zusammensetzung nach Anspruch 12, wobei die zusätzlichen desodorierenden Wirkstoffe unter Zinkpyrrolidoncarboxylat; Zinksulfat; Zinkchlorid; Zinklactat; Zinkgluconat; Zinkphenolsulfonat; 2,4,4'-Trichlor-2'-hydroxydiphenylether (Triclosan); 2,4-Dichlor-2'-hydroxydiphenylether; 3',4',5'-Trichlorsalicylanilid; 1-(3',4'-Dichlorphenyl)-3-(4'-chlorphenyl)-harnstoff (Triclocarban); 3,7,11-Trimethyldodeca-2,5,10-trienol (Farnesol); Cetyltrimethylammoniumsalzen, Cetylpyridiniumsalzen; Chlorhexidin und seinen Salzen; Diglycerylmonocaprat; Diglycerylmonolaurat; Glycerylmonolaurat; Salzen von Polyhexamethylenbiguanid ausgewählt sind.

14. Zusammensetzung nach Anspruch 12, wobei die schweißverhütenden Wirkstoffe unter Aluminiumhydroxychlorid, Aluminium Chlorohydrex, Aluminium Chlorohydrex PEG, Aluminium Chlorohydrex PG, Aluminium Dichlorohydrate, Aluminium Dichlorohydrex PEG, Aluminium Dichlorohydrex PG, Aluminium Sesquichlorohydrate, Aluminium Sesquichlorohydrex PEG, Aluminium Sesquichlorohydrex PG, Aluminiumsulfat, Aluminium Zirconium Octachlorohydrate, Aluminium Zirconium Pentachlorohydrate, Aluminium Zirconium Tetrachlorohydrate, Aluminium Zirconium Trichlorohydrate, Metallkomplexen mit einer Aminosäure ausgewählt sind.

15. Zusammensetzung nach Anspruch 14, **dadurch gekennzeichnet, dass** die mit den Metallkomplexen verwendete Aminosäure das Glycin ist.

16. Zusammensetzung nach einem der Ansprüche 12 bis 15, **dadurch gekennzeichnet, dass** die zusätzlichen desodorierenden Wirkstoffe und/oder schweißverhütenden Wirkstoffe in einer Menge von etwa 0,001 bis 20 Gew.-%, bezogen auf die gesamte Zusammensetzung, und vorzugsweise etwa 0,1 bis 10 Gew.-% enthalten sind.

17. Zusammensetzung nach einem der Ansprüche 1 bis 11, **dadurch gekennzeichnet, dass** sie keinen zusätzlichen desodorierenden Wirkstoff oder schweißverhütenden Wirkstoff enthält.

18. Zusammensetzung nach einem der Ansprüche 1 bis 17, **dadurch gekennzeichnet, dass** der kosmetisch akzeptable Träger ferner Wasser und/oder ein oder mehrere zusätzliche organische Lösungsmittel enthält.

19. Zusammensetzung nach einem der Ansprüche 1 bis 18, **dadurch gekennzeichnet, dass** sie als Wasser-in-Öl-Emulsion, Öl-in-Wasser-Emulsion oder Wasser-in-Öl-in-Wasser-Dreifachemulsion formuliert ist.

20. Zusammensetzung nach einem der Ansprüche 1 bis 19, **dadurch gekennzeichnet, dass** sie ferner ein oder mehrere kosmetische Adjuvantien enthält, die unter den Fettsubstanzen, Emollientien, beruhigenden Stoffen, Antioxidantien, Trübungsmitteln, Stabilisatoren, Schaumverhütungsmitteln, Hydratisierungsmitteln, Vitaminen, Parfums, Konservierungsmitteln, grenzflächenaktiven Stoffen, Füllstoffen, Maskierungsmitteln, Polymeren, Alkalisierungsmitteln oder Ansäuerungsmitteln, Parfums, Farbstoffen, Pigmenten, Verdickungsmitteln oder beliebigen anderen, gewöhnlich in der Kosmetik für diesen Anwendungstyp verwendeten Bestandteilen ausgewählt sind.

21. Zusammensetzung nach einem der Ansprüche 1 bis 20, **dadurch gekennzeichnet, dass** sie als Lotion, Creme oder fluides Gel vorliegt.

22. Aerosolvorrichtung, die aus einem Behälter, der eine Aerosolzusammensetzung enthält, die einerseits aus einer flüssigen Phase (oder Flüssigkeit), die mindestens einen C₁₋₄-Monoalkohol und Zinksalicylat in freier Form oder hydratisiert enthält, und mindestens einem Treibmittel besteht, wie sie in einem der vorhergehenden Ansprüche definiert sind, und einer Einrichtung zur Abgabe der Aerosolzusammensetzung besteht.

23. Vorrichtung nach Anspruch 22, **dadurch gekennzeichnet, dass** sich das Zinksalicylat, der oder die Monoalkohole und das oder die Treibmittel in dem Aerosolbehälter in der gleichen Abteilung oder in verschiedenen Abteilungen befinden können.

24. Verfahren zur Behandlung menschlicher Körpergerüche und insbesondere Achselgerüche unter Verwendung der in einem der Ansprüche 22 oder 23 beschriebenen Vorrichtung.
